# FASCICULE DE BREVET EUROPEEN

(11) **EP 0 685 442 B1**
(45) Date de publication et mention de la délivrance du brevet: **23.04.2003**
(21) Numéro de dépôt: 95401213.4
(22) Date de dépôt: 24.05.1995
(51) Int. Cl.: C07B 41/12, C07C 329/16, C07B 47/00, C07B 39/00, C07B 43/00, C07B 41/04

(54) **Réactif utile pour et procédé pour la synthèse d'ester et xanthate transestérifiable**
Reagenz und Verfahren zur Herstellung von Estern und umesterungsfähige Xanthate
Reagent and process for the Synthesis of esters and transesterifiable xanthates

(30) Priorité: 27.05.1994 FR 9406449
(43) Date de publication de la demande: 06.12.1995
(73) Titulaire: RHODIA CHIMIE, 92408 Courbevoie Cédex (FR)
(72) Inventeur: Boivin, Jean, F-91470 Forges-Les-Bains (FR); Henriet, Eric B., F-94000 Creteil (FR); Zard, Samir Z., F-91190 Gif-sur-Yvette (FR)
(74) Mandataire: Ricalens, François

(56) Documents cités:
- EP-A- 0 442 077
- US-A- 3 522 221
- 'Methoden der organischen Chemie (Houben-Weyl), 4. Auflage, Band IX' , STUTTGART, DE * page 817 - page 819 *
- TETRAHEDRON LETTERS, vol. 34, no. 17, 23 Avril 1993 OXFORD GB, pages 2763-2766, J. BOIVIN, ET AL.: 'A novel synthesis of 1,3-dithiol-2-ones from S-propargyl dithiocarbonates'
- JOURNAL OF THE AMERICAN CHEMICAL SOCIETY, vol. 113, no. 15, 17 Juillet 1991 WASHINGTON, DC US, pages 5874-5876, J. BOIVIN, ET AL.: 'Novel formal 3+2 annulation reaction based on S-proargyl dithiocarbonates (xanthates)'
- TETRAHEDRON LETTERS, vol. 32, no. 21, 20 Mai 1991 OXFORD GB, pages 2387-2390, K. HARANO, ET AL.: 'A simple one-pot synthesis of hydroisobenzothiophenes via three-step sequential pericyclic reactions of xanthates'
- POLYMER BULLETIN, vol. 21, no. 5, HEIDELBERG DE, pages 445-448, A. LE MINOR, ET AL.: 'Synthesis of S-dithiocarbonates with polymer-supported xanthates'
- JOURNAL OF ORGANIC CHEMISTRY, vol. 45, no. 1, 4 Janvier 1980 WASHINGTON DC US, pages 175-177, N.F. HALEY, ET AL.: 'Efficient and general synthesis of 1,3-dithioles'
- CHEMICAL ABSTRACTS, vol. 70, no. 17, 28 Avril 1969 Columbus, Ohio, US; abstract no. 77272t, M.I. ALIEV, ET AL.: 'Synthesis of some xanthates' page 276; & ZH. OBSHCH. KHIM., 1968, 38(12), 2622-2624,

## Description

La présente invention a pour objet un procédé utile pour la synthèse d'ester, un réactif permettant la mise en oeuvre de ce procédé et une famille de composés entrant dans la composition de ce réactif.
Elle concerne plus particulièrement une famille de xanthates transestérifiables leurs synthèses et leurs utilisations.
Les réactions de substitution nucléophile sont très utilisées mais certaines sont très difficiles à mener et l'on est toujours à la recherche de nouveaux groupes partants qui soient très réactifs et faciles à obtenir.

Par ailleurs un des problèmes les plus lancinant est souvent de transformer une fonction de type hydroxyle en groupe partant.
Il est en outre préférable de faire en sorte que ce groupe partant respecte la chiralité (soit en l'inversant soit en la maintenant telle quelle) du radical auquel est attaché ledit groupe partant.
C'est pourquoi un des buts de la présente invention est de fournir un groupe partant dont le départ respecte la chiralité de l'alcoyle (au sens étymologique) auquel il est attaché.

Un autre but de la présente invention est de fournir un procédé qui permette de former ce groupe partant.

Un autre but de la présente invention est de fournir un réactif qui permette de d'alcoyler les nucléophiles même faibles ou difficiles d'accès.

Un autre but de la présente invention est de fournir un réactif qui permette d'alcoyler les acides même mauvais nucléophiles (pKa au plus égal à 3 de préférence à 1) ou difficile d'accès (c'est à dire encombrés).

Un autre but de la présente invention est de fournir un réactif qui permette d'alcoyler les nucléophiles sans racémisation dudit alcoyle.

Un autre but de la présente invention est de fournir un réactif qui permette de d'alcoyler les nucléophiles avec inversion dudit alcoyle.

Ces buts, et d'autres qui apparaîtront par la suite, sont atteints au moyen d'un procédé pour alcoyler un nucléophile caractérisé par le fait que l'on soumet ledit nucléophile à l'action d'un xanthate propargylique d'alcoyle en présence d'un acide à une température comprise entre 0 et 300°C, et que le nombre de carbone dudit alcoyle est au moins égal à 3.
En général ; on fait agir un nucléophile le plus souvent à hydrogène mobile. C'est à dire qu'il est susceptible de libérer un ion hydrogène après la première phase de l'alcoylation.
Dans ce cas il peut être noté par la formule générale :

H - (X)

dans laquelle X est le reste d'un produit organique ou minéral.

Le xanthate de propargyle, quant à lui, répond avantageusement à la formule générale (I) :

R₃ - C ≡ C -CR₁R₂ - S- CS - Y-R (I)

dans laquelle R est le reste d'un alcool primaire, secondaire ou tertiaire et dont les éventuelles autres fonctions peuvent être protégées, et
où R₁, R₂ et R₃ semblables ou différents sont choisis parmi les atomes d'hydrogène et les radicaux hydrocarbonés à chaîne linéaire ou ramifiée contenant 1 à 20 atomes de carbone, avantageusement de 1 à 10 ;
où Y est un chalcogène.

Le document TETRAHEDRON LETTERS, vol. 34, no. 17, 1993, pages 2763-2766, J. BOIVIN, ET AL. décrit la cyclisation des xanthates propargyliques d'éthyle ou de méthyle et signale que lorsque l'on utilise un acide carboxylique simple comme agent de cyclisation on peut détecter des carboxylates de méthyle ou d'éthyle comme sous-produits.
Le document JOURNAL OF THE AMERICAN CHEMICAL SOCIETY, vol. 113, no. 15, 1991, pages 5874-5876, J. BOIVIN, ET AL., a également pour objet la cyclisation des xanthates propargyliques de méthyle.
Le document TETRAHEDRON LETTERS, vol. 32, no.21, 1991, pages 2387-2390, K. HARANO, ET AL., étudie la thermolyse de certains xanthates propargyliques.
Le document POLYMER BULLETIN, vol. 21, no. 5, 1989, pages 445-448, AL LE MINOR, ET AL., décrit la synthèse de certains xanthates propargyliques
Le document JOURNAL OF ORGANIC CHEMISTRY, vol. 45, no. 1, 1980, pages 175-177, N.F. HALEY, ET AL., décrit la décomposition de xanthates propargyliques sous l'action du brome avec formation d'un composé cyclique.
Le document CHEMICAL ABSTRACTS, vol. 70, no. 17, abstract no. 77272t, M.I. ALIEV, ET AL.,: "synthesis of some xanthates' page 276, & ZH. OBSHCH. KHIM., 1968, 38(12), 2622-2624, décrit la synthèse de divers xanthates propargyliques.
Le document EP-A-0 442 077 décrit un xanthate propargylique de vinyle et non d'alcoyle.
Le document US-A-3,522,221 cite certains xanthates d'alcoyle sans les décrire ni en donner la réactivité.
Il est préférable que la somme de carbones de R₁, R₂ et R₃ soit au plus égale à 30.
Ces radicaux hydrocarbonés à chaîne linéaire ou ramifiée contenant 1 à 20 atomes de carbone sont choisis parmi les alcoyles (alcoyle est pris dans son acception étymologique de reste d'un alcool après avoir ignoré la dite fonction alcool), y compris les aralcoyles et les aryles. Ces radicaux hydrocarbonés peuvent être substitués et porter diverses fonctions libres ou protégées.

Dans les formules précédentes c'est le radical R qui alcoylera le nucléophile tout en subissant le cas échéant l'inversion de Walden (le radical ayant subi l'inversion de Walden sera noté R').

Pour qu'une telle inversion puisse être détectée il faut que R ne soit pas primaire.

Par ailleurs si l'on désire utiliser l'invention pour alcoyler des nucléophiles encombrés il est préférable que R ne soit pas tertiaire, ou qu'à tout le moins un des sous-radicaux portés par le carbone portant la liaison "libre" soit méthyle. En outre certains xanthates d'alcool tertiaire sont peu stables et risquent d'évoluer vers des alcènes plus vite qu'ils n'alcoylent.

Enfin il convient de souligner que la présente invention est surtout utile pour alcoyler avec des radicaux relativement complexes c'est à dire des radicaux R dont le nombre de carbone est au moins égal à trois, de préférence à 5 atomes de carbone.
Le procédé peut toutefois être intéressant pour propyler ou butyler les nucléophiles réputés mauvais (c'est-à-dire ceux dont la nucléophilie est inférieure à celle des ions acétate, propionate, ou benzoate), ou encombrés.
Par "nucléophiles encombrés", on entend les nucléophiles dont l'un au moins des atomes situés en alpha, bêta ou gamma de l'atome nucléophile futur porteur du radical
R, est d'une part porteur d'au moins deux chaînes et d'autre part non porteur d'une double liaison. Dans le cas de fonctions portées par des aromatiques , on considérera comme encombrés seulement les noyaux dont au moins une des positions ortho est occupée.

Avantageusement R, (et donc R') est le reste d'un alcool, primaire ou secondaire, éventuellement protégé, choisi parmi :
· les radicaux dérivant des hydrocarbures monocycliques saturés, partiellement saturés ou insaturés, des hydrocarbures polycycliques ortho ou ortho et péricondensés, saturés, partiellement saturés ou insaturés, et pouvant être pontés, des hydrocarbures spiranniques, les hydrocarbures terpéniques, l'ensemble de ces hydrocarbures pouvant être mono ou poly substitués (par des atomes d'halogène ou par des radicaux alcoyle, alcoyloxyle, acyle ou alcoyloxycarbonyle éventuellement substitués, carboxyle éventuellement protégés, carbamoyle, alcoylcarbamoyle ou dialcoylcarbamoyle dont les radicaux alcoyles peuvent être eux-mêmes substitués, nitro, hydroxy éventuellement protégés, amino ou alcoylamino éventuellement protégés ou dialçoylamino, ou par des radicaux carbocycliques aliphatiques ou aromatiques, ou hétérocycliques, pouvant être eux-mêmes substitués),
· les hétérocycles mono ou polycycliques, saturés, partiellement saturés ou insaturés, contenant un ou plusieurs hétéroatomes choisis parmi l'azote, l'oxygène ou le soufre et éventuellement mono ou polysubstitués par des atomes ou radicaux tels que définis ci-dessus pour les hydrocarbures cycliques ;
· les radicaux alcoyles linéaires ou ramifiés contenant 3 à 10 atomes de carbone, éventuellement mono ou polyinsaturés et/ou mono ou polysubstitués par des atomes ou radicaux tels que définis ci-dessus pour les hydrocarbures cycliques ;
· le reste d'un macrolide ;
· le reste d'un sucre ;
· d'un alcaloïde ;
· d'un stéroïde.

A titre paradigmatique et non limitatif, les restes R d'alcool primaire ou secondaire ou tertiaire éventuellement protégés peuvent notamment être choisis parmi :
· par exemple ceux issus des hydrocarbures monocycliques ou polycycliques peuvent être choisis parmi des restes de dérivés de la famille des stéroïdes comme par exemple les restes du cholestérol ou ses dérivés, les restes du cholestanol ou ses dérivés, les restes de l'acide bétulinique éventuellement protégé ou ses dérivés. Les restes de l'acide cholique et de ses dérivés ;
· par exemple les hétérocycles mono ou polycycliques peuvent être choisis parmi les hétérocycles monocycliques contenant 5 à 7 chaînons tels que la pyrrolidine et ses dérivés, la pipéridine et ses dérivés, la proline ou ses dérivés, le thiazole ou ses dérivés, la morpholine et ses dérivés, la quinuclidine, un reste de la baccatine éventuellement protégé ou ses dérivés, la codéïne et ses dérivés ;
· par exemple les radicaux alcoyles linéaires ou ramifiés éventuellement mono ou polysubstitués peuvent être choisis parmi les radicaux propyle, i.propyle, n. Butyle, i.butyle, pentyle, néopentyle ... Ces chaînes pouvant être substituées (notamment par un ou plusieurs radicaux amino ou alcoylamino protégés ou non, ou par un dialcoylamino dont les parties alcoyle peuvent être éventuellement substituées ou former ensemble un hétérocycle pouvant comprendre d'autres hétéroatomes choisis parmi l'azote, I'oxygène ou le soufre, ou substituées par un ou plusieurs radicaux alcoyloxy ou alcoyloxycarbonyle dont la partie alcoyle peut être éventuellement substituée, ou substituées par un ou plusieurs radicaux carboxyles protégés, ou substituées par un ou plusieurs radicaux carbocycliques ou hétérocycliques mono ou polycycliques saturés, partiellement saturés ou insaturés et éventuellement eux même substitués.

A titre d'exemple le reste d'un macrolide peut être choisi parmi les synergistines des groupes A et B comme notamment les pristinamycines de type I et II, les virginiamycines S, la spiramycine, I'érythromycine, l'azithromycine.

A titre d'exemple le reste d'un sucre, y compris les nucléosides et nucléotides,
- d'un alcaloïde
- d'un stéroïde

A titre non limitatif le radical X peut être notamment choisis parmi :
- un reste d'acide organique carboxylique, par exemple un radical mono ou polycyclique saturé, partiellement saturé ou insaturé pouvant être éventuellement substitué [par un ou plusieurs radicaux hydroxyles éventuellement protégés, alcoyles, alcoyloxyles, alcoylthio, alcoyles ou alcoloxy ou alcoylthyio eux même substitués (notamment par un ou plusieurs atomes d'halogène ou radicaux hydroxyles éventuellement protégés, amino éventuellement protégés et éventuellement substitués, aminocarbonyles, carboxamido, carboxyles protégés, alcoyloxycarbonyles, hétérocyclyles ou carbocycles saturés ou insaturés et pouvant être eux mêmes substitués) ou par un ou plusieurs radicaux carboxyles protégés, alcoyloxycarbonyles, méthylène, oxo, ], ou un radical hétérocyclique mono ou polycyclique, saturé, partiellement saturé ou insaturé et comprenant un ou plusieurs hétéroatomes choisis parmi l'azote, l'oxygène ou le soufre et éventuellement substitué par des radicaux tels que définis ci-dessus pour les radicaux carbocycliques, ou un radical alcoyle contenant 1 à 10 atomes de carbone en chaîne linéaire ou ramifiée et éventuellement substitué par des radicaux tels que définis ci-dessus pour les radicaux carbocycliques.

A titre d'exemples :
- le reste d'un acide organique peut être notamment le reste d'un sucre dont les fonctions pouvant interférer avec la réaction sont préalablement protégées, les restes d'un acide nucléique dont les fonctions pouvant interférer avec la réaction sont préalablement protégées, le reste d'un acide dérivé de la famille des stéroïdes.
- un radical hétérocyclique à hydrogène mobile mono- ou poly-cyclique comme par exemple un radical hétérocyclique azoté dont les parties pouvant interférer avec la réaction sont préalablement protégées, et pouvant comprendre un ou plusieurs autres hétéroatomes choisis parmi l'azote, l'oxygène ou le soufre.

A titre d'exemple peuvent être cités les radicaux dérivés de tétrazolyle substitué (par exemple phényltétrazolyle), imidazolyle, triazolyle, phtalimido, hydroxy-1 phtalimido, oxo-2 pyrrolidinyle, succinimido, isoxazolyle.

D'une manière générale on peut citer comme H-(X) les acides "lato sensu" dont le pKa est dans la fourchette indiquée ci après pour les acides favorisant la transformation des xanthates propargyliques en une espèce constituant un excellent groupe partant, transformation qui conduira le groupe partant à former des composés du type dithiol-1,3 one-2 (cf "a novel synthesis of 1,3 dithiol-2-ones from S propargyl dithiocarbonates" de Jean Boivin, Eric Henriet , Catherine Tailhan et Samir Z. Zard tetrahedron letters Vol. 34, pp 2763-66 (1993)).
Parmi ces acides il convient de citer les acides que constituent les composés ayant un hydrogène en alpha d'au moins une fonction électroattractrice. Comme fonction électroattractrice on peut citer à titre de paradigme les sulfones, les carbonyles, les groupes nitro, les nitriles, et leurs équivalents.
Une autre famille de nucléophile préféré est constituée des acides halohydriques, les acides carboxyliques, phosphoriques, phosphoniques, phosphiniques, les arséniates, les hétérocycles mono ou polycycliques contenant 1 ou plusieurs hétéroatomes choisis parmi l'azote, le phosphore l'oxygène ou le soufre.
Lorsque les nucléophiles ne sont pas assez acides il faut adjoindre au nucléophile un acide plus fort (dans les limites de pKa ci dessous) afin de former l'espèce active.

Comme cela a été mentionné ci-dessus un des buts de la présente invention est de fournir un réactif utile pour alcoyler les nucléophiles.

Ce but est atteint au moyen d'un réactif à base de xanthate de propargyle ou de composés mesomères. II comporte pour addition successive ou simultanée :
◆ un xanthate propargylique d'alcoyle d'au moins 3 carbones
◆ au moins un acide de Brönsted dont le pKa est au plus égal à 12, de préférence à 10 ; ledit acide de Brönsted étant choisi parmi :
   · les acides de pKa au plus égal à 3 ;
   · les acides halohydriques, les acides carboxyliques, phosphoriques, phosphoniques, phosphiniques, les arséniates ;
   · les hétérocycles mono ou polycycliques contenant 1 ou plusieurs hétéroatomes choisis parmi l'azote, le phosphore, l'oxygène ou le soufre, et
   · parmi les composés ayant un hydrogène en alpha d'au moins une fonction électroattractrice.

Ce dernier composant peut constituer avec le substrat nucléophile un seul composé qui possède alors une fonction acide faible ou forte.
Avantageusement le réactif comporte un solvant. Les solvants sont de préférence aprotiques ou peu protiques et peu basiques pour éviter d'interférer avec l'acide et éventuellement le substrat.
Les solvants sont le plus souvent des dérivés aromatiques ou leurs mélanges. Le solvant joue le rôle de régulateur de température et l'on chauffe en général à l'ébullition.
Les solvants sont de préférence inertes vis-à-vis des réactifs et des substrats utilisés dans des conditions de mise en oeuvre de l'invention.

Le nucléophile peut être neutre, y compris zwitterionique, ou ionique. Il est porteur d'au moins une fonction, ou d'un atome, nucléophile. Cette fonction peut être neutre ou anionique.
Avantageusement le Xanthate propargylique d'alcoyle a pour formule la formule (I) suivante :

R₃ - C ≡ C -CR₁R₂ - S - CS - Y-R (I)

où R_{1'}, R₂ et R₃ sont des restes hydrocarbonés avantageusement d'au plus 10 atomes de carbone et où R contient au moins 3 atomes de carbone;
où R est un alcoyle;
où Y est un chalcogène.

Dans la présente description le terme alcoyle est pris dans son sens étymologique du reste d'un alcool après ignorance de la fonction alcool considérée.

La présente invention présentent surtout un intérêt pour synthétiser des composés complexes ce qui implique des radicaux R dont le nombre de carbone est supérieur à un, plus généralement à deux. Le départ du groupe issu du xanthate de propargyle se faisant avec inversion de Walden, la technique est particulièrement appropriée pour les synthèses chirales et d'une manière générale pour toute synthèse délicate.
Ce type de réaction présente aussi un avantage pour réaliser certains esters d'acide réfractaire à l'estérification, par exemple les d'acides encombrés en alpha ou en bêta de la de la fonction carboxylique.

Dans la présente description le terme ester est pris dans son sens le plus large à savoir un composé qui peut être obtenu par la condensation d'un acide même non oxygéné avec un alcool et ce avec élimination d'eau entre la fonction alcool et l'hydrogène de l'acide.
Par acide il faut entendre un acide de Brönstedt, ou en d'autre terme tout composé présentant au moins un hydrogène mobile, avantageusement plus acide que l'eau.
Plus spécifiquement le pKa est avantageusement au plus égal à 12, de préférence à 10.
Avantageusement Y est un chalcogène léger ; le second (soufre) ou de préférence le premier (Oxygène) de la série.
Il est préférable que la réaction ai lieu dans des conditions anhydres. C'est à dire que le la teneur en eau du milieu réactionnel soit au plus égal à 1 %, avantageusement à 1 ‰, de préférence à 10⁻⁴ (en masse).
La température de la réaction est avantageusement comprise entre 50 et 200°C de préférence entre 50 et 150°C. On utilise en général un solvant dont le reflux sous pression atmosphérique intervient à ces températures.
La synthèse des xanthates est bien connue de l'homme de métier et peut être réalisée par l'action d'alcoolate sur le sulfure de carbone, le sel obtenu étant condensé sur un halogénure ou un pseudohalogénure de propargyle éventuellement substitué.
Le radical R correspond au radical issu d'un alcool ou d'un thioalcool qui se greffe sur le nucléophile.

Dans la présente description, lorsque le ou les chiffres les plus à droite d'un nombre est
ou sont des zéros, ces zéros sont des zéros de position et non des chiffres significatifs, sauf bien entendu s'il en est précisé autrement.

Un autre but de la présente invention est de fournir une nouvelle famille de xanthate propargylique.

Un xanthate de propargyle de formule générale :

R₃ - C ≡ C - CR₁R₂ - S - CS - Y-R (I)

Dans laquelle R est un radical alcoyle, secondaire ou tertiaire, comportant au moins 5 atomes de carbone.
Dans laquelle R₁, R₂ et R₃, semblables ou différents, sont choisis parmi les atomes d'hydrogène et les radicaux hydrocarbonés, à chaîne linéaire ou ramifiée, contenant 1 à 20 atomes de carbone, avantageusement de 1 à 10 ; et
où Y est un chalcogène.

Les exemples suivants illustrent l'invention.

### PRODUITS DE DEPART

Les alcools utilisés pour la synthèse des xanthates des exemples suivants sont rassemblés dans le tableau ci-après.

| Alcools | Référence | origine |
|---|---|---|
| L'octyn-1-ol 3- | 1-0 | commerce |
| Phenyl-1-butyn-1-ol-3 | 2-0 | méthode de Luche 1 |
| cyclohexen-1-butyn-1-ol-3 | 3-0 | méthode de Luche 1 |
| phenyl-1-propyn-1-ol-3 | 4-0 | méthode de Luche 1 |
| méthyl-4-ene-5-hexyn-2-ol-1 | 6-0 | commerce |
| butyn-1-ol-3 | 7-0 | commerce |
| méthyl-2-butyn-3--ol 1 | 13-0 | commerce |
| triphenylméthoxy-1-propanol-2 | 14-0 | voir ci-dessous |
| Diacétone glucose | 15-0 | commerce |
| L'alcool benzylique | 16-0 | commerce |
| 3-méthyl-3-oxetaneméthanol | 17-0 | commerce |
| Codéine | 18-0 | commerce |
| Quinudidinol-3 S (+) | 19-0 | commerce |
| Quinuclidinol-3 | 19-0 | commerce (mélange racémique) |
| cyclohexen-1-propyn-1-ol-3 | 23-0 | méthode de Luche ¹ |
| phenyl-1-propyn-2-ol-1 | 24-0 | commerce |
| Menthol (+,-) | 25-0 | commerce |
| L'octanol-2 | 26-0 | commerce |
| L'alcool phényléthylique | 27-0 | commerce |

Le premier chiffre de la référence utilisée dans la présente description est repris dans la suite des exemples pour indiquer la présence du radical issu de ces alcools.

### II-2 Méthode au n-Butyllithium

### II-2.1 Préparation du propargyl-cholesteryl dithiocarbonate

Dans un tricol avec arrivée d'argon et ampoule à brome, on introduit 2,84 mmoles de cholestérol (1,1 g, M = 386,66) dans 50 ml de THF sec. On y ajoute quelques cristaux de 2,2'- bipyridine. On additionne du n-Butyllithium 2,5 M dans l'hexane jusqu'à coloration rouge persistante (2,84 mmol, M = 64,06, d = 0,88, V = 2,5 ml). On laisse agiter puis on ajoute 13 mmoles de CS₂ ( 1g, 0,8 ml). On agite à température ambiante pendant 20 minutes. On ajoute alors 15 mmoles de chlorure de propargyle (1,123 g, 5 eq, M = 74,51). La réaction est suivie par C. C. M. On extrait du dichlorométhane et une solution d'eau salée. On sèche avec Na₂SO₄ et on évapore le dichlorométhane.

On récupère après colonne (éluant : éther de pétrole) le xanthate de cholestérol. Le rendement est d'environ 80 % en xanthate et allène.

### II-2.2 Préparation du propargyl-cholestanyl dithiocarbonate 12-0

Dans un tricol avec arrivée d'argon et ampoule à brome, on introduit 7,72 mmoles de cholestanol (3 g, M = 388,66) dans 50 ml de THF sec.
On y ajoute quelques cristaux de 2,2'-bipyridine.
On additionne du n-Butyllithium 2,5 M dans l'hexane jusqu'à coloration rouge persistante (M = 64,06, d = 0,88, V = 3 ml).
On laisse agiter puis on ajoute 54 mmoles de CS₂ (7 eq, 4,1 g, 3,3 ml, M = 76).
On agite à température ambiante pendant 20 minutes.
On ajoute alors 38,6 mmoles de chlorure de propargyle (2,9 g, 5 eq, M = 74,51). La réaction est suivie par C.C.M. (CHROMATOGRAPHIE SUR COUCHE MINCE).
On extrait avec du dichlorométhane et une solution d'eau salée.
On sèche avec Na₂SO₄.
On évapore le dichlorométhane.
On récupère après colonne (éluant : éther de pétrole) le xanthate de cholestanol. Le rendement est quantitatif en xanthate et en allène.

### II-3 Méthode à l'hydrure de potassium KH

Dans un tricol (que l'on a taré) sous argon, on introduit une suspension d'hydrure de potassium dans l'huile.
On ajoute du pentane sec ; on agite pendant une minute et on pipette le pentane surnageant.
On réitère ceci trois fois lavant ainsi l'hydrure de son huile.
On sèche alors sous un fort courant d'argon.
On obtient alors une poudre d'hydrure sec.
On pèse le ballon et on en déduit la quantité X de KH (M = 40,11) mise dans le tricol.
On ajoute alors V ml de THF sec.
On se place à 78°C.
On ajoute alors au moyen d'une ampoule à brome, Y mmol d'alcool.
On surveille l'avancement de la réaction en mesurant l'hydrogène qui se dégage. Lorsqu'il n'y a plus d'hydrogène se dégageant, on se place à 0°C et on ajoute 2 à 3 eq de CS₂ (V' ml, M = 76).
On laisse ainsi trente minutes la température remonter à celle de la pièce.
On ajoute alors W g de chlorure de propargyle (M = 74,51, 3 à 4 eq).
On verse (sauf cas particulier) dans une solution d'eau et d'acide citrique. On extrait à l'éther et on sèche au sulfate de sodium. Après une colonne rapide, on récupère le xanthate propargylique recherché.

On a les résultats suivants :

| **XANTHATES** | **14 - 1** | **15 - 1** | **17 - 1** | **18 - 1** | **19 - 1 r** | **19 - 1 s** | **25 - 1** | **26 - 1** | **27 -** |
|---|---|---|---|---|---|---|---|---|---|
| **KH, X mmol** | 56,3 | 47,1 | 82,0 | 3,73 | 6,73 | 13,1 | 24,9 | 35,6 | 39,0 |
| **THF sec, V ml** | 20 | 20 | x | 5 | 5 | 10 | 15 | 20 | 30 |
| **Alcool, Y mmol** | 56,3 | 47,1 | 90 | 3,4 | 7 | 13,1 | 26,2 | 37,4 | 42,5 |
| **CS2, V' ml** | 5 | 5,7 | 25 | 0,7 | 1,3 | 2,5 | 4,5 | 6,5 | 7,0 |
| **Chlor. de prop., W g** | 7,5 | 7,5 | 15,2 | 0,9 | 1,0 | 2 | 3,8 | 5,3 | 5,9 |
| **Rendement, %** | 92 | 96 | 2,6 | 23,9 | 90 | 88 | 62 | > 95 | > 95 |
| **Notes** | | | * | | | | | | |

| | | | | | | | | | |
|---|---|---|---|---|---|---|---|---|---|
| *** Dans cette expérience, le solvant utilisé était le CS**_{**2**} **lui-même. Le faible rendement est vraisemblablement explicable par une forte volatilité du produit. On a dû distiller à la fois CS**_{**2**} **et l'éther de l'extraction avec un piège à carboglace.** | | | | | | | | | |

### IV- Préparation d'ester simples

### IV-1 Réaction thermique de l'acide correspondant avec un S-propargylxanthate

Le S-(Propyn-1-yl-3)-O-Néopentyl dithiocarbonate, ***10-0.***
Une solution de X mmol de xanthate et de Y mmol d'acide dans V ml de chlorobenzène
ou toluène selon les cas est chauffée à reflux pendant 5 heures environ.
On suit la réaction par C.C.M. (chromatographie sur couche mince) On évapore le solvant (cas du toluène) à l'évaporateur rotatif. Dans certains cas, l'ester cristallise dès cette étape ; on filtre et recristallise alors ce dernier. Sinon, on effectue une colonne (on se débarrasse ainsi du solvant dans le cas du chlorobenzène) et on recristallise l'ester.

Les conditions opératoires sont indiquées dans le tableau ci-dessous. En plus de l'ester, on obtient à chaque expérience le mélange des deux isomères de la 1,3-dithiol-2-one correspondant au xanthate utilisé.

| **ESTER** | **X, mmol** | **Y, mmol** | **V, ml** | **Durée** |
|---|---|---|---|---|
| 10 A | 0,92 | 1,38 | 4 | 5 h |

On a les résultats suivants :

| **ACIDE** | **ESTER** | | **SOLVANT** | **Rdt%** | **Recrist.** |
|---|---|---|---|---|---|
| | **ref.** | **TYPE** | | | |
| Sucre | 10 A | Néopentylique | Toluène | 93 | |

### V- Préparation d'esters plus élaborés

### V-1 Benzoates du cholestérol

On porte à reflux dans le toluène (5 ml), 1,1 mmol de xanthate (**propargyl-cholesteryl dithiocarbonate)** (0,547 g, M = 500,66) et 1,5 eq d'acide benzoïque (1,65 mmol, 0,2 g, M = 122) pendant 5 heures. Après évaporation du solvant, on effectue une colonne pour récupérer le benzoate-3β, 11 A ainsi que le benzoate-5β, 11A' résultant du phénomène dit i-stéroïde. Le rendement global est de 65 %. On a comme sous-produits les 1,3-dithiol-2-ones ***10-3*** et ***10-4*** ainsi qu'une trace de 2-cholestène.

### V-2 Esters du cholestanol

### VI-2,1 Mode opératoire

Dans un ballon, on introduit X mmol de xanthate de cholestanol ***12-0*** (M = 502,68) et Y mmol d'acide dans V ml de toluène.
On porte à reflux pendant environ 5 heures.
On évapore le solvant, on sèche sous azote et on effectue une colonne pour récupérer l'ester.

On a les résultats suivants :

| **Acide** | **Ester** | **X, mmol** | **Y, mmol** | **V, ml** | **Rdt %** |
|---|---|---|---|---|---|
| Benzoique | 12 A | 0,628 | 1,2 | 4 | 55,75 |
| Benzoïque | 12 A bis | 0,302 | 0,6 | 4 | 78,6 |
| galacturonique | 12 B | 0,24 | 0,18 | 4 | 97 |
| Coumaliaue | 12 I | 0,503 | 0,553 | 4 | 77,1 |
| Thiobenzoique * | 12 J | 0,491 | 0,54 | 5 | 50 |

| | | | | | |
|---|---|---|---|---|---|
| *** Pour l'ensemble de ces expériences, le rendement complémentaire est en 2-cholestène sauf dans le cas de l'acide thiobenzoique où l'on obtient que 24 % en 2-cholestène (fraction 1). On note aussi dans la fraction 2 la présence d'un cholestène (M = 370) qui peut être le 3-cholestène.** | | | | | |

### V-5 Ester à partir du 3-méthyl-3-oxétaneméthanol

Dans un ballon, on introduit 2,0 mmol de xanthate ***17-1*** (M = 216) et 2,0 mmol d'acide N-acéthyl tryptophane dans 3 ml de toluène.
On porte à reflux pendant environ 5 heures
On évapore le solvant, sèche sous azote et on effectue une colonne pour récupérer l'ester.
On l'obtient avec une rendement de 79 %.

### V-6 Benzoate de codéine

Dans un ballon, on introduit 0,237 mmol de xanthate 18-1 (M = 413) et 0,300 mmol d'acide benzoique dans 2,5 ml de toluène
On porte à reflux pendant environ 5 heures.
On évapore le solvant, on alcalinise et on extrait avec un mélange dichlorométhane /-éther de pétrole (50/50). On sèche au sulfate de sodium et on effectue une colonne pour récupérer l'ester avec un rendement qui reste à déterminer.

### V-7 Benzoates de la 3-quinuclidinol

### V-7.1 En partant d'un mélange racémique de 3-quinuclidinol

Dans un ballon, on introduit 3,45 mmol du mélange racémique de xanthate ***19-1*** (M = 240) et 6,9 mmol d'acide benzoique dans 4 ml de toluène.
On porte à reflux pendant environ 5 heures. On évapore le solvant et on lave avec une solution de NaHCO₃ saturée.
On extrait à l'éther.
On sèche sur sulfate de sodium et on effectue une colonne pour récupérer. Le rendement est de 36 %.

### V-7.2 En partant de la 3-quinuclidinol S(+)

Dans un ballon, on introduit 0,9 mmol du xanthate ***19-1*** S (M = 240) et 1,8 mmol d'acide benzoïque dans 3 ml de toluène.
On porte à reflux pendant environ 6 heures.
On évapore le solvant et on lave avec une solution de NaHCO₃ saturée.
On extrait à l'éther.
On sèche sur sulfate de sodium et on effectue une colonne pour récupérer l'ester (éluant : éther : dichlorométhane triéthylamine = 45 / 50 / 5).
Le rendement est de 63 %.
On récupère aussi en première fraction de la colonne le mélange des 1,3-dithiol-2-ones.

Afin de vérifier l'inversion, on réalise un témoin (benzoate non inversé) en mettant 50 mg de quinuclidinol S (+) dans 2 ml de pyndine et en ajoutant 0,1 ml de PhCOCI (M = 140,5, d = 1,211). Après une semaine, on verse dans NaHCO₃ et on extrait l'éther.

### V-8 Benzoate du menthol (+ , -)

Dans un ballon,on introduit 1,85 mmol de xanthate ***25-1*** (M = 270) et 1,48 mmol d'acide benzoïque dans 3,5 ml de toluène.
On porte à reflux pendant environ 6 heures.
On évapore le solvant, sèche sous azote et on effectue une colonne pour récupérer l'ester.
Le rendement est de 47 %.
On récupère aussi 10 % de xanthate de départ et le mélange des 1,3-dithiol-2-ones.

Les deux isomères du menthol au départ se trouvent être en proportions équatorial /-axial = 3/2 (déterminée par R.M.N. ¹H). Celles-ci se conservent lors de la préparation du xanthate.

Par contre sur le spectre proton du benzoate, on peut constater l'inversion de ce rapport. En hydrolysant le mélange des deux benzoates (e/a = 2/3), on a récupérer du menthol avec des proportions inversées par rapport à celui utilisé au départ.

### VI Réactions apparentées

### VI-1 Préparation de phosphates et phosphites

Dans un ballon muni d'un condensateur à reflux et d'un agitateur, on introduit X mmol de xanthate et Y mmol de diphénylphosphate (M = 250,19) ou de diméthylphosphite (M = 110;05, d = 1,2) dans V ml de toluène.

On porte à reflux. Après traitement (colonne), on a les résultats suivants :

| Réactif | Xanth. | X | Y | V | Rdt | Cholestène |
|---|---|---|---|---|---|---|
| Phosphate | 12 - 0 | 0,56 | 0,62 | 4 | 28,00 % | 68,90 % |
| Phosphate | 26 - 0 | 1,86 | 3,19 | 3,5 | 18,00 % | |
| Phosphite | 12 - 0 | 0,36 | 0,65 | 3 | 27,30 % | 58,30 % |

### VI-2 Préparation d'halogénures du cholestane

Dans un ballon muni d'un condensateur à reflux et d'un agitateur, on introduit X mmol de xanthate de cholestanol ***12-0*** (M = 502,68) et Y mmol de 4-chloropyridine, HCI (pour le composé chloré), de trihydrofluorure de triéthylamine (pour le composé fluoré) ou de Et₃N ; Hl (pour le composé iodé) dans 3 à 4 Ml de toluène. On porte à reflux.

Après traitement (colonne) on a les résultats suivants :

| Réactif | Produit | X, mmol | Y, mmol | 2- Cholestène | Halogénure |
|---|---|---|---|---|---|
| "HCI" | 12 G | 0,157 | 0,345 | 14,13 % | 59,20 % |
| "HF" | 12 H | 0,219 | 0,438 | 38,20 % | 60,35 % |
| "HI" | 12 L | 0,53 | 1,6 | 3,50 % | 58,4 %* |

| | | | | | |
|---|---|---|---|---|---|
| *** Dans le cas de l'ioduration, le détail des rendements est en fait :** | | | | | |

| | |
|---|---|
| **iodure** | 58,4 % **(fractions 1 et 2)** |
| **2-cholestène (par R.M.N.** ^{**1**}**H)** | **3,50 % (fraction 1)** |
| **xanthate 12-0** | **35,16 %** |
| **cholestanol** | **3,30 %** |

### Préparation de Et₃N:HI

Dans un ballon contenant 0,31 mol de triéthylamine (50 ml),on ajoute goutte à goutte 0,104 mol de Hl, 67 % (20 g). On filtre et on récupère le réactif quantitativement.

### VII Alcoylation de l'imidazole par le S-propargyl-O-octyldithiocarbonate

Dans un ballon contenant 8 ml de toluène, on introduit 8,4 mmol d'imidazole (0,57 g, M = 68,08, 1,2 eq ; pKa = 14) et 1,4 mmol d'acide camphre sulfonique, monohydrate (0,34 g, M = 250,32, 0,2 eq).
On évapore par ébullition le toluène jusqu'à ce qu'il en reste 4 ml. Ainsi par azéotropie, on déshydrate le milieu réactionnel.

On ajoute alors 0,7 mmol de triéthylamine (0,07 g, M = 101,1g, 0,1 eq) et 7 mmol de xanthate d'octyle ***26-1*** (1,65 g, M = 236,1 eq).
On porte au reflux du toluène pendant 5 heures.
On évapore le toluène et on effectue un rapide rapide passage sur colonne de silice.
On a les rendements suivants :
- 13,8 % en N-(octyloxycarbonnyl)-imidazole ,
- 25,5 % N-(octyl-2)-imidazole.

## Revendications

1. Procédé pour alcoyler un nucléophile **caractérisé par le fait que** l'on soumet ledit nucléophile à l'action d'un xanthate propargylique d'alcoyle en présence d'un acide à une température comprise entre 0 et 300°C, et que le nombre de carbone dudit alcoyle est au moins égal à 3.

2. Procédé selon la revendication 1, **caractérisé par le fait que** ledit alcoyle présente un nombre de carbone au moins égal à 5.

3. Procédé selon les revendications 1 et 2, **caractérisé par le fait qu'**il consiste à faire agir un nucléophile à hydrogène mobile de formule générale :
H - (X)
dans laquelle X est le reste d'un produit organique ou minéral sur un xanthate de propargyle répondant à la formule générale :
R₃ - C ≡ C -CR₁R₂ - S - CS - Y-R (I)
dans laquelle R est le reste d'un alcool primaire, secondaire ou tertiaire et dont les éventuelles autres fonctions peuvent être protégées ;
où R₁, R₂ et R₃, semblables ou différents, sont choisis parmi les atomes d'hydrogène et les radicaux hydrocarbonés à chaîne linéaire ou ramifiée contenant 1 à 20 atomes de carbone, avantageusement de 1 à 10 ; et
où Y est un chalcogène.

4. Procédé selon la revendication 3, **caractérisé par le fait que** lesdits radicaux hydrocarbonés, à chaîne linéaire ou ramifiée, contenant 1 à 20 atomes de carbone sont choisis parmi les alcoyles, y compris les aralcoyles et les aryles.

5. Procédé selon les revendications 3 et 4, **caractérisé par le fait que** l'on obtient un composé de formule R - X.

6. Procédé selon les revendications 3 à 5, **caractérisé par le fait que** R est chiral et que l'on obtient un composé de formule R' - X où R' est le radical R ayant subi l'inversion de Walden.

7. Procédé selon les revendications 3 à 6, **caractérisé par le fait que** dans la formula (I), R est le reste d'un alcool, primaire ou secondaire, éventuellement protégé, choisi parmi :
• les radicaux dérivant des hydrocarbures monocycliques saturés, partiellement saturés ou insaturés, des hydrocarbures polycycliques ortho ou ortho et péricondensés, saturés, partiellement saturés ou insaturés et pouvant être pontés, des hydrocarbures spiranniques, les hydrocarbures terpéniques, l'ensemble de ces hydrocarbures pouvant être mono ou poly substitués (par des atomes d'halogène ou par des radicaux alcoyle, alcoyloxyle, acyle ou alcoyloxycarbonyle éventuellement substitués, carboxyle éventuellement protégé, carbamoyle, alcoylcarbamoyle ou dialcoylcarbamoyle dont les radicaux alcoyles peuvent être eux-mêmes substitués, nitro, hydroxy éventuellement protégés, amino ou alcoylamino éventuellement protégés ou dialcoylamino, ou par des radicaux carbocycliques aliphatiques ou aromatiques, ou hétérocycliques, pouvant être eux-mêmes substitués) ;
• les hétérocycles mono ou polycycliques, saturés, partiellement saturés ou insaturés, contenant un ou plusieurs hétéroatomes choisis parmi l'azote, l'oxygène ou le soufre et éventuellement mono ou polysubstitués par des atomes ou radicaux tels que définis ci-dessus pour les hydrocarbures cycliques ;
• les radicaux alcoyles linéaires ou ramifiés contenant 3 à 10 atomes de carbone, éventuellement mono ou polyinsaturés et/ou mono ou polysubstitués par des atomes ou radicaux tels que définis ci-dessus pour les hydrocarbures cycliques ;
• le reste d'un macrolide ;
• le reste d'un sucre ;
• d'un alcaloïde ;
• d'un stéroïde.

8. Procédé selon les revendications 3 à 7, **caractérisé par le fait que** le radical X est choisi parmi un reste d'acide organique carboxylique.

9. Procédé selon les revendications 3 à 7, **caractérisé par le fait que** le produit de formule générale H-X est choisi parmi les acides halohydriques, les acides carboxyliques, phosphoriques, phosphoniques, phosphiniques, les arséniates, les hétérocycles mono ou polycycliques contenant 1 ou plusieurs hétéroatomes choisis parmi l'azote, le phospore, l'oxygène ou le soufre.

10. Utilisation d'un xanthate propargylique (-R) pour la préparation d'un produit de structure R-X, dans laquelle R est un radical alcoyle d'au moins 3 atomes de carbone et X est un reste d'un nucléophile porteur d'hydrogène de formule H - X.

11. Utilisation, selon la revendication 10, d'un xanthate de propargyle de formule générale (I) :
R₃ - C ≡ C -CR₁R₂ - S - CS - Y-R (I)
dans laquelle R₁, R₂ et R₃, semblables ou différents, sont choisis parmi les atomes d'hydrogène et les radicaux hydrocarbonés à chaîne linéaire ou ramifiée contenant 1 à 20 atomes de carbone, avantageusement de 1 à 10 ;
où Y est un chalcogène ;
et où R est le reste d'un alcool secondaire éventuellement protégé, pour la préparation d'un produit de formule générale :
R'-X
dans laquelle R' est le reste dudit alcool secondaire, avec inversion de la configuration.

12. Un xanthate de propargyle de formule générale :
R₃ - C ≡ C - CR₁R₂ - S - CS - Y-R (I)
dans laquelle R est un radical alcoyle, secondaire ou tertiaire, comportant au moins 5 atomes de carbone.
Dans laquelle R₁, R₂ et R₃, semblables ou différents, sont choisis parmi les atomes d'hydrogène et les radicaux hydrocarbonés à chaîne linéaire ou ramifiée contenant 1 à 20 atomes de carbone, avantageusement de 1 à 10 ; et
où Y est un chalcogène.

13. Xanthate selon la revendication 12, **caractérisé par le fait que** R est chiral.

14. Réactif comportant pour addition successive ou simultanée un xanthate propargylique d'alcoyle d'au moins 3 carbones et au moins un acide de Brönsted dont le pKa est au plus égal à 12, de préférence à 10 ; ledit acide de Brönsted étant choisi parmi
• les acides de pKa au plus égal à 3 ;
• les acides halohydriques, les acides carboxyliques, phosphoriques, phosphoniques, phosphiniques, les arséniates ;
• les hétérocycles mono ou polycycliques contenant 1 ou plusieurs hétéroatomes choisis parmi l'azote, le phosphore, l'oxygène ou le soufre et
• parmi les composés ayant un hydrogène en alpha d'au moins une fonction électroattratrice.

15. Réactif selon la revendication 14, **caractérisé par le fait que** ladite fonction électroattractrice est choisie parmi les sulfones, les carbonyles, les groupes nitro, les nitriles.

16. Réactif selon les revendications 14 et 15 **caractérisé par le fait qu'**il comporte un solvant aprotique.

## Claims

1. Process for alkylating a nucleophile, **characterized in that** the said nucleophile is subjected to the action of a propargyl alkyl xanthate in the presence of an acid at a temperature of between 0 and 300°C and **in that** the carbon number of the said alkyl is at least equal to 3.

2. Process according to Claim 1, **characterized in that** the said alkyl exhibits a carbon number at least equal to 5.

3. Process according to Claims 1 and 2, **characterized in that** it consists in reacting a nucleophile comprising mobile hydrogen of general formula:
H-(X)
in which X is the residue of an organic or inorganic product, with a propargyl xanthate corresponding to the general formula:
R₃-C≡C-CR₁R₂-S-CS-Y-R (I)
in which R is the residue of a primary, secondary or tertiary alcohol, the other possible functional groups of which can be protected;
where R₁, R₂ and R₃, which are alike or different, are chosen from hydrogen atoms and linear- or branched-chain hydrocarbonaceous radicals comprising 1 to 20 carbon atoms, advantageously from 1 to 10; and
where Y is a chalcogen.

4. Process according to Claim 3, **characterized in that** the said linear- or branched-chain hydrocarbonaceous radicals comprising 1 to 20 carbon atoms are chosen from alkyls, including aralkyls, and aryls.

5. Process according to Claims 3 and 4, **characterized in that** a compound of formula R-X is obtained.

6. Process according to Claims 3 to 5, **characterized in that** R is chiral and **in that** a compound of formula R'-X, where R' is the R radical having undergone Walden inversion, is obtained.

7. Process according to Claims 3 to 6, **characterized in that**, in the formula (I), R is the residue of an optionally protected primary or secondary alcohol chosen from:
• radicals deriving from saturated, partially saturated or unsaturated monocyclic hydrocarbons, saturated, partially saturated or unsaturated, ortho- or ortho- and pericondensed polycylic hydrocarbons which can be bridged, spiro hydrocarbons or terpene hydrocarbons, it being possible for all these hydrocarbons to be mono- or polysubstituted (by halogen atoms or by alkyl, alkyloxyl, acyl or alkyloxycarbonyl radicals which are optionally substituted, optionally protected carboxyl radicals, carbamoyl, alkylcarbamoyl or dialkylcarbamoyl radicals, the alkyl radicals of which can be themselves substituted, nitro radicals, optionally protected hydroxyl radicals, optionally protected amino or alkylamino radicals or dialkylamino radicals, or by heterocyclic or aliphatic or aromatic carbocyclic radicals which can be themselves substituted);
• saturated, partially saturated or unsaturated, mono- or polycyclic heterocycles comprising one or more heteroatoms chosen from nitrogen, oxygen or sulphur which are optionally mono- or polysubstituted by atoms or radicals as defined above for the cyclic hydrocarbons;
• linear or branched alkyl radicals comprising 3 to 10 carbon atoms which are optionally mono- or poly-unsaturated and/or mono- or polysubstituted by atoms or radicals as defined above for the cyclic hydrocarbons;
• the residue of a macrolide;
• the residue of a sugar;
• the residue of an alkaloid;
• the residue of a steroid.

8. Process according to Claims 3 to 7, **characterized in that** the X radical is chosen from an organic carboxylic acid residue.

9. Process according to Claims 3 to 7, **characterized in that** the product of general formula H-X is chosen from hydrohalic acids, carboxylic, phosphoric, phosphonic or phosphinic acids, arsenates, or mono- or polycyclic heterocycles comprising 1 or more heteroatoms chosen from nitrogen, phosphorus, oxygen or sulphur.

10. Use of a propargyl (-R) xanthate for the preparation of a product of structure R-X in which R is an alkyl radical of at least 3 carbon atoms and X is a residue of a hydrogen-carrying nucleophile of formula H-X.

11. Use according to Claim 10 of a propargyl xanthate of general formula (I):
R₃-C≡C-CR₁R₂-S-CS-Y-R (I)
in which R₁, R₂ and R₃, which are alike or different, are chosen from hydrogen atoms and linear- or branched-chain hydrocarbonaceous radicals comprising 1 to 20 carbon atoms, advantageously from 1 to 10;
where Y is a chalcogen;
and where R is the residue of an optionally protected secondary alcohol, for the preparation of a product of general formula:
R'-X
in which R' is the residue of the said secondary alcohol, with inversion of the configuration.

12. A propargyl xanthate of general formula:
R₃-C≡C-CR₁R₂-S-CS-Y-R (I)
in which R is a secondary or tertiary alkyl radical comprising at least 5 carbon atoms,
in which R₁, R₂ and R₃, which are alike or different, are chosen from hydrogen atoms and linear- or branched-chain hydrocarbonaceous radicals comprising 1 to 20 carbon atoms, advantageously from 1 to 10; and
where Y is a chalcogen.

13. Xanthate according to Claim 12, **characterized in that** R is chiral.

14. Reactant comprising, for successive or simultaneous addition, a propargyl alkyl xanthate, the alkyl having at least 3 carbons, and at least one Brönsted acid, the pKa of which is at most equal to 12, preferably to 10; the said Brönsted acid being chosen from
• acids with pKa values at most equal to 3;
• hydrohalic acids, carboxylic, phosphoric, phosphonic or phosphinic acids, or arsenates;
• mono- or polycyclic heterocycles comprising one or more heteroatoms chosen from nitrogen, phosphorus, oxygen or sulphur, and
• compounds having a hydrogen in the alpha position with respect to at least one electron-withdrawing functional group.

15. Reactant according to Claim 14, **characterized in that** the said electron-withdrawing functional group is chosen from sulphones, carbonyls, nitro groups or nitriles.

16. Reactant according to Claims 14 and 15, **characterized in that** it comprises an aprotic solvent.

## Patentansprüche

1. Verfahren zur Alkylierung eines Nucleophils, **dadurch gekennzeichnet, daß** man das Nucleophil der Wirkung eines Alkyl-Propargyl-Xanthogenats in Gegenwart einer Säure bei einer Temperatur zwischen 0 und 300 °C unterwirft und daß die Anzahl der Kohlenstoffatome des Alkyls mindestens 3 ist.

2. verfahren nach Anspruch 1, **dadurch gekennzeichnet, daß** das Alkyl eine Anzahl der Kohlenstoffatome von mindestens 5 aufweist.

3. Verfahren nach Anspruch 1 und Anspruch 2, **dadurch gekennzeichnet, daß** ein Nucleophil mit beweglichem Wasserstoff der allgemeinen Formel
H- (X)
worin X der Rest eines organischen oder anorganischen Produktes ist, mit einem Propargyl-Xanthogenat, das der allgemeinen Formel
R₃ - C ≡ C -CR₁R₂ - S - CS - Y-R (I)
entspricht, worin R der Rest eines primären Alkohols, sekundären Alkohols oder tertiären Alkohols ist, bei dem die etwaigen anderen Funktionen geschützt sein können; worin R₁, R₂ und R₃, die gleich oder verschieden sind, unter Wasserstoffatomen und linearen oder verzweigten Kohlenwasserstoffgruppen, die 1 bis 20 Kohlenstoffatome, vorzugsweise 1 bis 10 Kohlenstoffatome enthalten, ausgewählt werden und worin Y ein Chalcogen ist, reagieren gelassen wird.

4. Verfahren nach Anspruch 3, **dadurch gekennzeichnet, daß** die linearen oder verzweigten Kohlenwasserstoffgruppen, die 1 bis 20 Kohlenstoffatome enthalten, unter Alkylgruppen, einschließlich der Aralkyle und Aryle, ausgewählt werden.

5. Verfahren nach Anspruch 3 oder Anspruch 4, **dadurch gekennzeichnet, daß** man eine Verbindung der Formel R-X erhält.

6. Verfahren nach den Ansprüchen 3 bis 5, **dadurch gekennzeichnet, daß** R chiral ist und daß man eine Verbindung der Formel R'-X erhält, worin R' die Gruppe R ist, die der Walden-Umkehr unterworfen wurde.

7. Verfahren nach den Ansprüchen 3 bis 6, **dadurch gekennzeichnet, daß** in der Formel (I) R der Rest eines primären oder sekundären Alkohols, der gegebenenfalls geschützt ist, ist, der ausgewählt wird unter:
- Gruppen, die von monocyclischen gesättigten, teilweise gesättigten oder ungesättigten Kohlenwasserstoffen, von polycyclischen ortho- oder ortho- und para-kondensierten, gesättigten, teilweise gesättigten oder ungesättigten Kohlenwasserstoffen, die eine Brückenbindung haben können, von Spiro-Kohlenwasserstoffen, von Terpen-Kohlenwasserstoffen abgeleitet sind, wobei die Gesamtheit dieser Kohlenwasserstoffe mono- oder polysubstituiert sein kann (durch Halogenatome oder durch gegebenenfalls substituierte Alkyl-, Alkyloxy-, Acyl- oder Alkyloxycarbonyl-Gruppen, gegebenenfalls geschütztes Carboxyl, Carbamoyl, Alkylcarbamoyl oder Dialkylcarbamoyl, in denen die Alkyl-Gruppen selbst substituiert sein können, Nitro, gegebenenfalls geschütztes Hydroxy, Amin oder Alkylamino, die gegebenenfalls geschützt sind, oder Dialkylamino oder durch aliphatische oder aromatische carbocyclische Reste oder heterocyclische Reste, die selbst substituiert sein können);
- mono- oder polycyclischen gesättigten, teilweise gesättigten oder ungesättigten Heterocyclen, die ein oder mehrere Heteroatome, ausgewählt aus Stickstoff, Sauerstoff oder Schwefel, enthalten und gegebenenfalls durch Atome oder Gruppen, wie sie oben für die cyclischen Kohlenwasserstoff definiert sind, monooder polysubstituiert sind;
- linearen oder verzweigten Alkylgruppen, die 3 bis 10 Kohlenstoffatome enthalten, gegebenenfalls mono- oder polyungesättigt und durch die Atome und Gruppen, wie sie oben für die cyclischen Kohlenwasserstoffe definiert sind, mono- oder polysubstituiert sind;
- dem Rest eines Macrolids;
- dem Rest eines Zuckers;
- einem Alkaloid;
- einem Steroid.

8. Verfahren nach den Ansprüchen 3 bis 7, **dadurch gekennzeichnet, daß** die Gruppe X unter Resten organischer Carbonsäuren ausgewählt wird.

9. Verfahren nach den Ansprüchen 3 bis 7, **dadurch gekennzeichnet, daß** das Produkt der allgemeinen Formel H-X unter den Halogenwasserstoffsäuren, den Carbonsäuren, den Phosphorsäuren, den Phosphonsäuren, den Phosphinsäuren, den Arsenaten, mono- oder polycyclischen Heterocyclen, die ein oder mehrere Heteroatome, ausgewählt unter Stickstoff, Phosphor, Sauerstoff und Schwefel, enthalten, ausgewählt wird.

10. Verwendung eines Propargyl-Xanthogenats (-R) zur Herstellung eines Produktes der Struktur R-X, in der R eine Alkylgruppe mit mindestens drei Kohlenstoffatomen und X ein Rest eines nucleophilen Wasserstoffträgers der Formel H-X ist.

11. Verwendung nach Anspruch 10 eines Propargyl-Xanthogenats der allgemeinen Formel (I) :
R₃ - C ≡ C -CR₁R₂ - S - CS - Y-R (I)
in der R₁, R₂ und R₃, die gleich oder unterschiedlich sind, unter Wasserstoffatomen und linearen oder verzweigten Kohlenwasserstoffgruppen, die 1 bis 20 Kohlenstoffatome, vorzugsweise 1 bis 10 Kohlenstoffatome, enthalten, ausgewählt sind;
worin Y ein Chalcogen ist; und
worin R der Rest eines sekundären, gegebenenfalls geschützten, Alkohols ist, zur Herstellung eines Produktes der allgemeinen Formel
R'-X
worin R' der Rest des genannten sekundären Alkohols mit Inversion der Konfiguration ist.

12. Propargyl-Xanthogenat der allgemeinen Formel:
R₃ - C ≡ C -CR₁R₂ - S - CS - Y-R (I)
in der R eine sekundäre oder tertiäre Alkylgruppe ist, die mindestens 5 Kohlenstoffatome umfaßt,
worin R₁, R₂ und R₃, die gleich oder unterschiedlich sind, unter Wasserstoffatomen und linearen oder verzweigten Kohlenwasserstoffgruppen, die 1 bis 20 Kohlenstoffatome, vorzugsweise 1 bis 10 Kohlenstoffatome, enthalten, ausgewählt werden; und
worin Y ein Chalcogen ist.

13. Xanthogenat nach Anspruch 12, **dadurch gekennzeichnet, daß** R chiral ist.

14. Reagens, das zur sukzessiven oder gleichzeitigen Zugabe ein Alkyl-Propargyl-Xanthogenat mit mindestens 3 Kohlenstoffatomen und mindestens eine Brönsted-Säure, deren pKa höchstens 12, vorzugsweise 10 ist, umfaßt, wobei die Brönsted-Säure unter
- Säuren mit einem pKa von höchstens 3,
- Halogenwasserstoffsäuren, Carbonsäuren, Phosphorsäuren, Phosphonsäuren, Phosphinsäuren. Arsenaten,
- mono- oder polycyclischen Heterocyclen, die ein oder mehrere Heteroatome, ausgewählt aus Stickstoff, Phosphor, Sauerstoff und Schwefel, enthalten und
- unter den Verbindungen, die ein Wasserstoff in alphaStellung zu mindestens einer elektronenanziehenden Funktion haben, ausgewählt ist.

15. Reagens nach Anspruch 14, **dadurch gekennzeichnet, daß** die elektronenanziehende Funktion unter Sulfonen, Carbonylen, Nitro-Gruppen, Nitrilen ausgewählt ist.

16. Reagens nach Anspruch 14 und Anspruch 15, **dadurch gekennzeichnet, daß** es ein aprotisches Lösungsmittel umfaßt.
